# EUROPEAN PATENT SPECIFICATION

(11) **EP 2 947 634 B1**
(45) Date of publication and mention of the grant of the patent: **25.12.2019**
(21) Application number: 15397524.8
(22) Date of filing: 21.05.2015
(51) Int. Cl.: G08B 21/04, A61B 5/11, A61B 5/00, G06K 9/00, G16H 40/20, G16H 50/30

(54) **ARRANGEMENT, METHOD AND COMPUTER SOFTWARE PRODUCT FOR MONITORING SAFETY IN ELDERLY CARE**
ANORDNUNG, VERFAHREN UND COMPUTERSOFTWAREPRODUKT ZUR ÜBERWACHUNG DER SICHERHEIT BEI DER ALTENPFLEGE
DISPOSITIF, PROCÉDÉ ET PRODUIT LOGICIEL INFORMATIQUE PERMETTANT DE SURVEILLER LA SÉCURITÉ LORS DE SOINS AUX PERSONNES ÂGÉES

(30) Priority: 21.05.2014 FI 20145456
(43) Date of publication of application: 25.11.2015
(73) Proprietor: Eagle Vision Management Oy, 90440 Kempele (FI)
(72) Inventor: Kuronen, Pentti, 40270 Palokka (FI); Hannula, Manne, 90440 Kempele (FI); Kuronen, Lauri, 00140 Helsinki (FI)
(74) Representative: Berggren Oy, Helsinki & Oulu

(56) References cited:
- US-A1- 2008 186 189
- US-A1- 2012 253 233
- TARIK YARDIBI ET AL: "Gait characterization via pulse-Doppler radar", PERVASIVE COMPUTING AND COMMUNICATIONS WORKSHOPS (PERCOM WORKSHOPS), 2011 IEEE INTERNATIONAL CONFERENCE ON, IEEE, 21 March 2011 (2011-03-21), pages 662-667, XP031865754, DOI: 10.1109/PERCOMW.2011.5766971 ISBN: 978-1-61284-938-6

## Description

This invention concerns a system for monitoring safety in elderly care, which system comprises a sensor system, which is configured to monitor at least one elderly person, a processing system comprising a data storage, which processing system is configured to collect, process, and store the data which said sensor system has collected, which data includes at least time and location. The invention also concerns a method and a computer software product for monitoring safety in elderly care.

### PRIOR ART

In elderly home care there is a need for increasing of safety. The need for increase of safety is especially important from two points of views. First, ability to live independently despite of decreased functional impairment is an important value for elderly from quality-of-life perspective. All tools which support this objective are important. Second, it is evident that because of general trend about increasing imbalance between elderly care need and related resources, automated technological resources are needed to support missing of human resources or to catalyze the effectiveness of usage of limited human resources in care work.

### Current sensor technology and automated alarm solutions for elderly care

Maturity of sensor technology for assessment of health is in a good level; for example publications US2008186189 US2008319309, US2008319326, US2008312519, EP1952291, WO2008085179, US2008167566, US2009005700, WO2008154800, WO2008148067, MXPA06010576, KR20080089497, CN201127601, NZ543267, US2008300499 and GB2448716 give an insight into some current solutions of different kinds of measurements. In the field of elderly care a lot of research has been done for example about applicability of movement or accelerometer sensor technology for elderly care purposes; publications US2014026664 and CN203204787 show a method and a device concerning automatic fall detection for elderly people. Fall detection and its analyses are good examples about focused applications to increase safety in elderly care with the help of technology. Even trousers which alarm about falling have been developed as described in publication CN201398476.

Analyses of health status of the elderly people have been studied also in a wider perspective than only by single measurements and their interpretation like in fall detection cases. In publication CN102708658 there is reported an automated alarming system for detecting unusual conditions of daily routine, which can be applied e.g. in elderly care to produce automated alarms even in cases where the person to be monitored is unable to alarm autonomously. Further, besides occurrence of specific signs, the absence of some information may be utilizable in automated alarms applications. The publication DE3830655 shows one solution for producing automated alarms e.g. based on absence of specific signals. Also indirect assessment of daily routines has been proposed to apply in publication KR20110022171. In this solution usage of energy in the house was analyzed to monitor elderly peoples' living.

Analyses of daily routines or typical behavior of elderly peoples for safety purposes is not a new invention. Already in the 1980's an automated time sequence based alarm system was reported in publication GB2149168 to produce an automated alarm if the person has not flushed the toilet during the last 24 hours. The rapid development of information and communication technology has boosted development of applications especially during the last ten years. Many systems comprising sensors and communication tools which can be applied for remote health surveillance of people have been reported, e.g. in publication CN201710473. Further, automated systems for monitoring independently living persons who may require operational assistance have been developed, as reported in publication US2002171551.

### Risk management approaches in elderly care

Safe and security concepts risk management. In this document term "safe" means experience or feeling of the subject in trusting to be free from a threat of danger, harm, or risk. Correspondingly, the term "security" is used to describe procedures and devices for creating a better safety situation of people or a better security of objects. This invention concerns principally the term safe in elderly care, by integration of required elements, including security issues.

In publication KR20080050993 an apparatus and method for fall detecting is presented in light of providing safe life for elderly peoples. The feeling of safe and safety is important; in the publication JP2005278765 a system which supports the safe life of elderly people is presented, by using monitoring of posture, breathing, heart rate, and the like measurements. Above those technology-oriented approaches there are methods to evaluate risks of diseases or infections as illustrated in publication US2012112883, to be applied e.g. in infection spread control in pandemic cases. The predictive health outcome modeling in disease management has been done in publication EP0917078, by executing risk analyses on the basis of different kinds of data sources. Also risk management about focused applications, like in specific surgical operations, has been developed as reported in CN102789536.

It is necessary to emphasize that advanced risk management has been studied and implemented a very limited amount in healthcare compared to e.g. industry or finance fields.

Role of human factors in risk management and work process development is important. A challenging point is that human factors cannot be determined according to exact principles compared to machines, devices, software, or the like tools, which also are part of the same working processes.

### How to improve dispersed elderly care management by further processing findings from "swarming concept"

There is a well-known and usable tactical way to be used e.g. in air warfare tactics which is known as a swarming concept. The idea contains the principles of concentrating abilities and force in a dispersed environment, exemplified with the following references:
"The systematic pulsing of force and/or fire by dispersed, internetted units, so as to strike the adversary from all directions". *Reference: John Arquilla & David Ronfeldt Swarming and the Future of Conflict"*
"Maneuver involved the convergent attack of several semiautonomous (or autonomous) units on a target force in some particular place". *Reference: Sean J. A. Edwards Swarming on the Battlefield. Past, Present, and Future. Published 2005 by the RAND Corporation."*

Thus, together with improved situational awareness (SA) and ways to process huge amount of information in cloud solutions, to handle and process it by innovative networked communication solutions, it is possible to improve essentially organizational challenges of elderly care. The novel ways to apply long used practices of flight safety and warfare management tactics to elderly care management might improve essentially management processes which have been fairly conservative for a long time and urgently need new innovations supported by technological solutions. It is evident that theoretical application equalities about benefits of swarming can be found from both asymmetric warfare field and asymmetric situation in elderly care with continuous increase of requirements and continuous decrease of human resources available.

### Problems in current solutions

The solutions enhancing the safety and security concepts and processes for elderly care have faced several problems. So far, it has been typical to implement sensor technology to measure features which correlate with safety and security. However, the utilization of the data and collected information as integrated system concept has been inadequate. For example, emerging amount of data from wireless sensors cannot be adequately utilized or possibilities of cloud service based data systems cannot be turned to support concretely elderly care. Further, the basic problem has been lack of definitive approach of measured actions, which relates to working process management of elderly care. The referred publications illustrate case examples about actions from sensor data to alarms. The requirements of safe and safety, as part of risk management, however, are not fulfilled by that kind of approaches. Apparently, the security information by the sensor solutions has not been turned into production of safety for users.

### Lack of situational awareness and inadequate information and communication resources

Lacking of situational awareness in elderly care management is a significant problem. The available solutions provide some possibilities e.g. for determining the current situation of the elderly care by help of computer based elderly care management information and communication technology (ICT) systems, or typically, for making statistical analyses or reports afterwards. However, the time domain understanding is missing about projecting the near future. With the current systems it has not been possible to predict reliably and applicable form scenarios of the near future. Correspondingly, simulation of upcoming situations in light of risk management seems not to be possible. Missing understanding of optimal usage of human resources decreases cost-effectiveness. The statement might be that the current situation analysis is not adequately connected to predicting the future and needs of resources. That means lacking of the situation awareness.

A significant problem relating to the situation awareness is inadequate utilization of time window beginning from the very early symptoms of increasing risks decreasing performance of ageing individuals. The preventing and rehabilitative actions should be executed optimally during this time frame. The practical problem has been that there is so much information produced by many organizations that the fragmented systems are unable to produce sufficient image of the information. Rapid communication and the related decision making is important in hectic situations of elderly care, weakness in the management in this sector may have substantial negative economic effect on the care organization.

The general problem in the known risk management related systems in elderly care seems to be the lack of analyzing resources and data for projecting the future actions. Also, this is incompatible with the risk management thinking, where preventive actions are important. The inadequate applications of ICT and analyses processing resources are problems when thinking of system approach of management of elderly care.

### Problems producing security solutions but not safety environment

The poor communication about risk management between elderly and the care professionals is also a problem in the current management environment. The elderly people themselves are not appropriately informed about safety arrangements. For example, tailored application for automated identification of falling might be used. However, the kind of approach provides only a very limited solution for increasing safety if the data analyzing and service system elements are not integrated.

Besides, inadequate awareness of risk management as well for elderly people as elderly care organizations is a reality. The experience of assessment of risks would be important for the personnel of elderly care organization in their complex working environment. Also, the regulations of authorities for elderly care must be obeyed. What great benefits for preventive safety actions and innovative working procedures would be available by using automated and integrated data management by SBA type systems.

### SUMMARY

The object of the invention is to solve the many problems associated with the present and scattered solutions of elderly care management.

The method named Safety Ball Analysis (SBA) is a novel tool for a practical implementation of supporting technologies for the benefit of the elderly. The SBA includes elements from sensor technology, communication solutions, information analyzes, risk management and modeling, interaction between automated systems and elderly care resources, services and interfaces to elderly care organizations and to other possible parties, like relatives.

There are significant benefits and possibilities available from the novel method of SBA. The SBA is able to integrate sensor information technologies, it is able to "mine and analyze big data" and produce the novel and automated SA to clients who need the information. The reporting of the SA is easy to scale and focused delivery of SA information can be automatically done, according to organization's need. This kind of way of collecting information and delivering it to those needing the applicable information has long time been used in complicated safety dependable environments like in air traffic.

The improved SA can be achieved by SBA method which has abilities to utilize performances of data analyzes and modeling of comprehensive risk management. The model of improved SA will be applied here in novel system and innovative management solution for elderly care.

The final solution of SBA concept will be the improved and automatically documented processes to enhance safety and security as a part of daily life of the elderly and the responsible care organizations. Also, continuous and further development of the working processes will be possible by using the information based on continuous registration and storing.

### Characterization of the invention

The object of the invention is a solution, by which the disadvantages and drawbacks of the prior art can be diminished. Particularly, the object of the invention is a solution, by which the safety in the elderly care can be increased and monitoring it will be more efficient.

The objects according to the invention are achieved by a system, method and computer program product, characterized in what is disclosed in the independent claims. Some preferred embodiments of the invention are disclosed in the dependent claims.

The leading idea of the invention is to gather data from at least one elderly person and process this data in a way that the system recognizes the imminent and approaching danger situations and informs about those to the elderly care personnel.

According to one embodiment of the invention, a system for monitoring safety in elderly care comprises a sensor system, which is configured to monitor at least one elderly person, a processing system comprising a data storage, which processing system is configured to collect, process and store the data which said sensor system has collected, which data includes at least time and location. According to one preferred embodiment of the invention, the processing system further comprises an individual data storage location that is configured to store data collected from an individual elderly person, a current situation processing element which is configured to analyze current situation of the elderly person based on the information available in the data storage, and to produce variables that describe the status of the elderly person, and a situation awareness processing element which is configured to store prediction pattern or patterns and to read said collected data and said prediction patterns and form an information set or sets based on them and predict upcoming situations of the elderly person based on the information in the individual data storage location and the information set or sets, and to produce variables that describe the future status of the elderly person. The processing system is configured to calculate one or more information items based on the variables produced by the current situation processing element or the situation awareness processing element or both, which each information item corresponds a risk value, and to alarm if the risk value of an information item exceeds some predetermined value. The system comprises an information element which provides the elderly person with information about the care process around him/her and the information is based on the calculations of the processing system.

In an embodiment of the system according to the invention the information items having approximately the same time, i.e. which are recorded about at the same time, form a group and the processing system is configured to form a ball shaped visual presentation based on said group. This ball shaped visual presentation is called a ball. The information items having a low risk value are situated near the center of the said ball, the information items having an increased risk value are situated to deviate from the center of the ball, and the information items causing the alarm are situated outside of the boundary of the ball. This visual presentation can be displayed on an appropriate medium like on a computer screen, phone, or similar.

In a second embodiment of the system according to the invention, the balls with different times are displayed sequentially on a time axis.

In a third embodiment of the system according to the invention, balls based on the information items based on the variables produced by the situation awareness processing element describing a future prediction of the safety of the elderly person.

In a fourth embodiment of the system according to the invention, an individual risk management table is stored in the individual data storage location and the individual risk management table contains at least a risk description column having one or more risk descriptions and to each risk description is connected one or more resolution limited parameters and each resolution limited parameter has several states and said individual risk management table is used to calculate the risk values of the information items.

In a fifth embodiment of the system according to the invention, the resolution limited parameters are configured to be scaled by the safety needs of the individual elder.

In a sixth embodiment of the system according to the invention, the information produced by the information element is a message that describes the situational awareness characteristics of the processing system which situational awareness characteristics are related to one or more information items.

In a seventh embodiment of the system according to the invention, the information indicates a risk level of the elderly person or action the system has executed or will execute or both the risk level and the action, and the risk level is calculated from a risk value. In an eighth embodiment of the system according to the invention, the information indicates that the situation of the elderly person is normal.

According to one embodiment of the invention, a method for monitoring safety in elderly care uses a system that comprises a sensor system, which is configured to monitor at least one elderly person, a processing system comprising a data storage, which processing system is configured to collect, process, and store the data which said sensor system has collected, which data includes at least time and location. According to one preferred embodiment of the invention the system further comprises an information element and the method comprises steps where the sensor system monitors the elderly person and gathers data, the processing system produces variables that describe the status of the elderly person based on the data the sensor system has gathered, the processing system calculates one or more information items based on the variables produced and each information item corresponds a risk value and if any of the risk values exceeds some predetermined value, the processing system gives an alarm, and the information element provides the elderly person with information about the care process around him/her.

In an embodiment of the method according to the invention the information items having approximately the same time are displayed as a group and the processing system forms a ball-shaped visual presentation where the boundary of the ball represents the predetermined value that triggers the alarm and the information items having a low risk value are situated near the center of the said ball, the information items having an increased risk value are situated to deviate from the center of the ball, and the information items causing the alarm are situated outside of the boundary of the ball.

In a second embodiment of the method according to the invention in the system has been stored prediction pattern or patterns and an information set or sets are formed based on said prediction pattern or patterns and the data the sensor system has gathered, and the processing system produces variables that describe the future status of the elderly person and based on these variables, predictive information items are calculated.

In a third embodiment of the method according to the invention the information element produces a message that describes the situational awareness characteristics of the processing system which situational awareness characteristics are related to one or more information items.

In a fourth embodiment of the method according to the invention the information provided by the information element indicates at least that the alarm is sent.

All the above-described steps of monitoring safety can also be realised with computer program commands which are executed in a suitable general or special-purpose processor. The computer program commands can be stored in a computer-readable media, such as a data disk or a memory, from where the processor can retrieve said computer program commands and execute them. The references to computer-readable media can for example also contain special components, such as programmable USB Flash memories, logic arrays (FPLA), application-specific integrated circuits (ASIC), and signal processors (DSP).

### DESCRIPTION OF THE FIGURES

- Figure 1: shows an example of the elements of the infrastructure of SBA;
- Figure 2: shows an example of the principle of SBA in a graphical form;
- Figure 3: shows utilization example of SBA and the features in time domain;
- Figure 4: shows an example of preventive perspective of SBA in risk management of elderly care;
- Figure 5: shows an example of the meaning of the comprehensive risk management principle of SBA and the role of mathematical modeling;
- Figure 6: shows an example of the role of cumulative information of SBA; and
- Figure 7: shows an example of dynamical features of application ("swarming") of SBA in elderly care management.

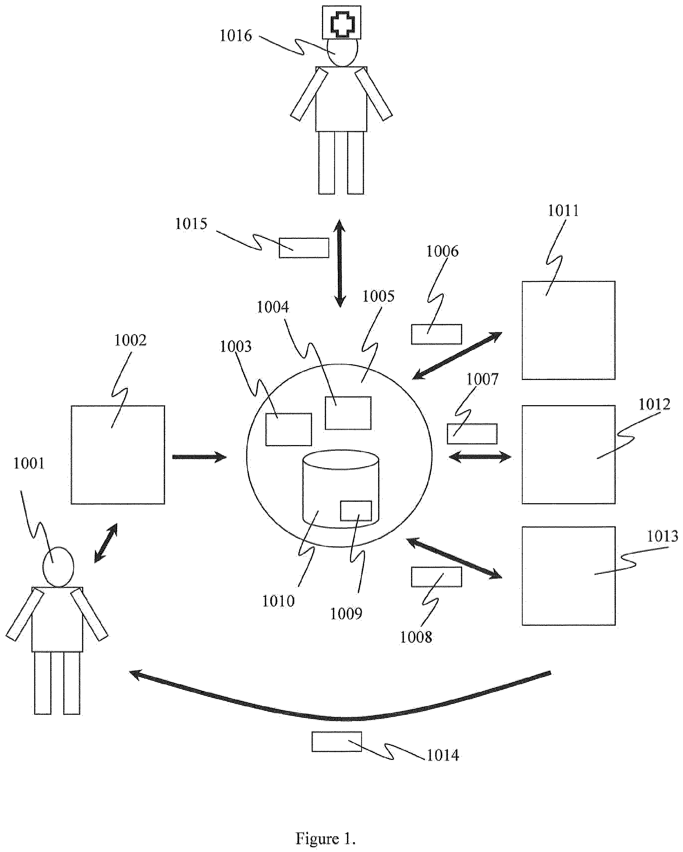

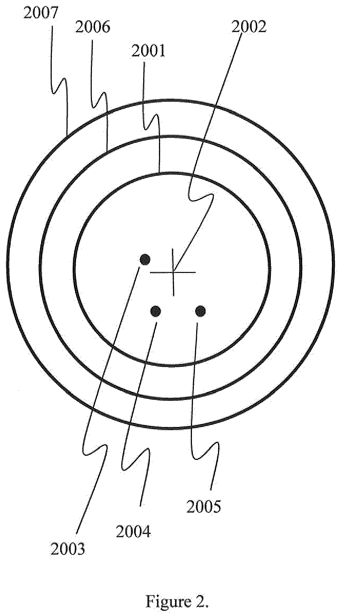

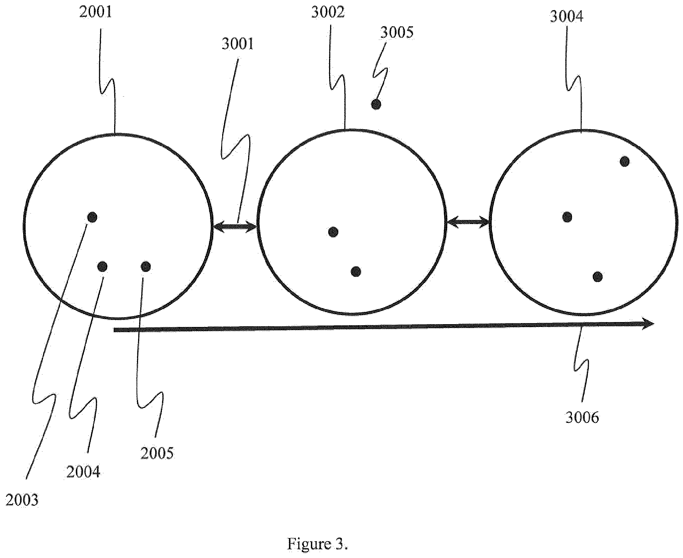

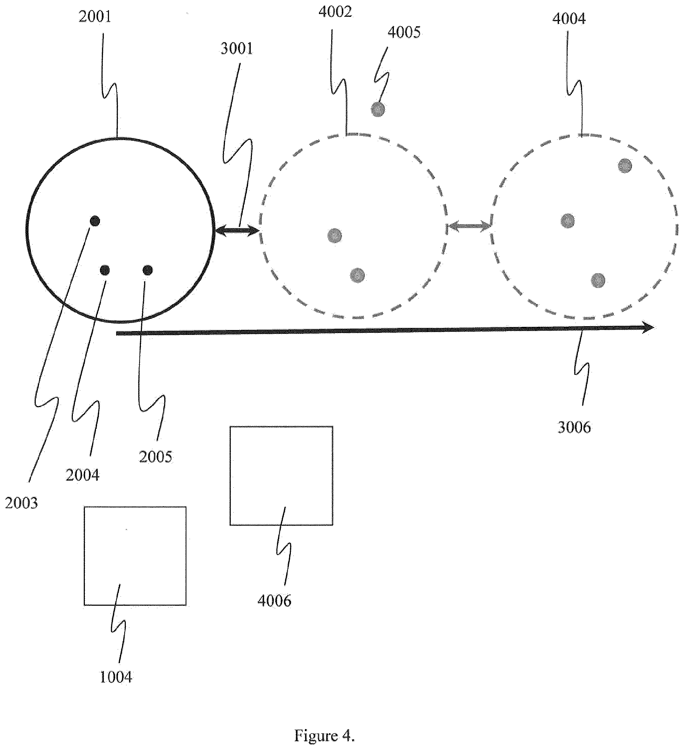

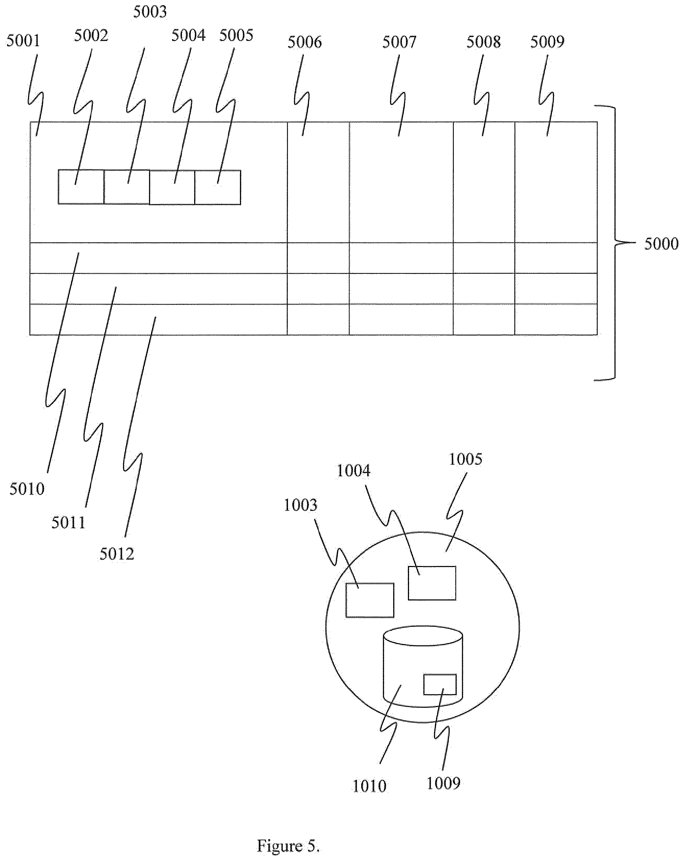

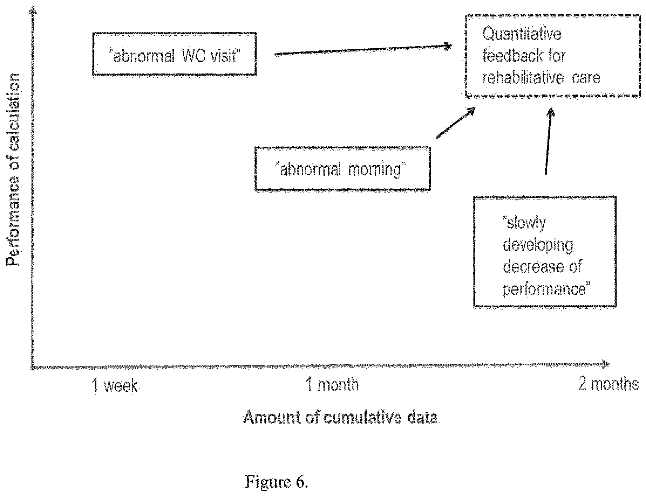

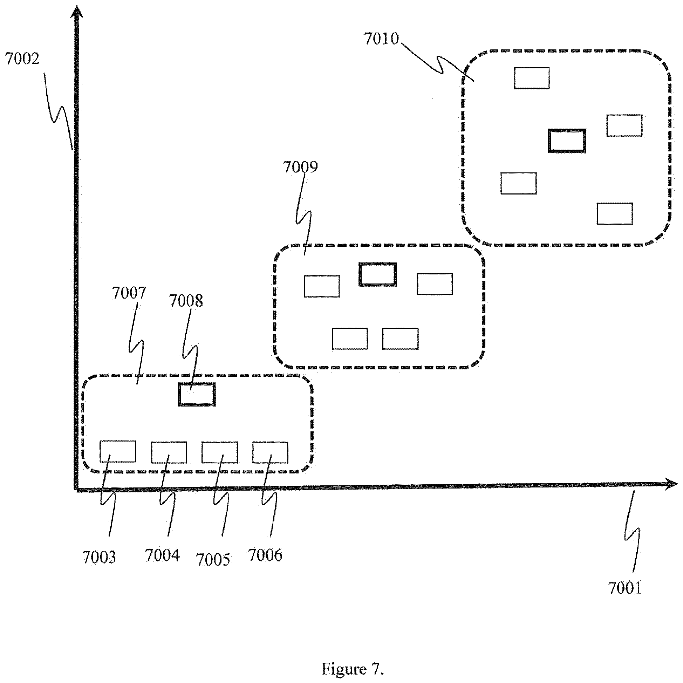

The invention is described by means of some examples with reference to Figure 1 which presents the essential elements of the infrastructure of SBA. Figure 2 illustrates principle of SBA in a graphical form. Figure 3 describes utilization of SBA and the features in time domain. Figure 4 illustrates a preventive perspective of SBA in risk management of elderly care. Figure 5 describes the meaning of the comprehensive risk management principle of SBA and the role of mathematical modeling. Figure 6 shows the role of cumulative information of SBA. Figure 7 exemplifies dynamical features of application ("swarming") of SBA in elderly care management.

The embodiments in the following description are given as examples only and a person skilled in the art can carry out the basic idea of the invention also in some other way than what is described in the description. Though the description may refer to a certain embodiment or embodiments in several places, this does not mean that the reference would be directed towards only one described embodiment or that the described characteristic would be usable only in one described embodiment. The individual characteristics of two or more embodiments may be combined and new embodiments of the invention may thus be provided.

### DETAILED DESCRIPTION OF FIGURES

### The basic infrastructure of elderly care according to invention

The background infrastructure of SBA consists of a sensor system 1002, connected to the elderly person 1001 with the appropriate means depending on the sensor system, which are a direct contact or wireless sensors. The sensor system 1002 is further connected to a processing system 1005 including a data storage 1010, which further includes individual data storage location 1009 for individual elderly people 1001. The data storage 1010 forms the basis of an "SBA-library", which can be arranged to form entities of selected and definitive information of the data storage. This can be used for further management, organizing and utilization of the data for the comprehensive processing in the processing system 1005. The processing system 1005 includes specific processing systems; current situation processing element 1003 and situational awareness processing element 1004. The current situation processing element 1003 includes means for analyzing current situation of the elderly person 1001 with the help of all latest information available in data storage 1010. The situational awareness processing element 1004 includes corresponding elements to predict possible upcoming situations of the elderly person 1001, by using information in an individual data storage location 1009 as a basis but also other appropriate information in a data storage location 1010 to make predictions. Together, the elements 1003 and 1004 form a complementary part for risk management analyzes. In addition, the previous parts of the processing system include means for connection sensor data and analyses to elderly care resource 1016, remote specialist service 1011, to customer information system 1012 and to education and training system 1013. In principal, the elderly care resource 1016 is a nurse, but it can be also another kind of human resource of elderly care, e.g. a relative of the elderly person. The remote specialist service 1011 can be e.g. a remote physician service, the customer information system 1012 can be e.g. a customer information database and an education system 1013 can be a mobile phone integrated education application. This will provide the elderly care resource 1016 with instructions as to how to utilize proper available services in the current situation of elderly 1001.

### Operation of the system analysis center and its communication with elements of the elderly care processes

The processing system 1005 communicates with service elements 1011, 1012, and 1013 on the basis of configurations done in the data storage 1010. Some parts of the configurations are the same for elderly people under the same elderly care organization. The individual configurations are done by using information in individual data storage location 1009.

The communication between the elderly person 1001, processing system 1005, elderly care resource 1016, and service elements 1011, 1012, and 1013 happens with the help of information elements 1006, 1007, 1008, 1014, and 1015. The purpose of information element 1006 is to inform the processing system 1005 about the currently available remote specialist services and correspondingly to inform the remote specialist service about required actions relating to elderly care needs e.g. of an elderly person 1001. The information processing system 1005 is responsible for the required information to the corresponding information element about the current situation of the elderly person 1001, including data from the sensor system 1002 in a suitable form to be utilizable for remote consultancy, and possible information which can be found from the customer information system 1012, as well possible existing supporting information from the education system 1013. Further, the purpose of the information element 1007 is to provide information processing system 1005 with the required customer information about the elderly person, as defined in the individual data storage location 1009. Moreover, information element 1008 typically carries information from the processing system 1005 toward the education system 1013 about the needs of training or education concerning the overall situation in the elderly care on the basis of data in data storage 1010, and correspondingly the education system gives information to the processing system 1005 about specific guidance e.g. to be used for the current remote consultancy from the remote specialist service 1011 to the elderly person 1001, or relating instructions to the elderly care resource 1016. Further, the information element 1015 may include required combination of information around the processing system 1005 e.g. about the current service need about the elderly person 1001. Finally, information element 1014 provides the elderly person 1001 with important and appropriate information about the care process around him/her. This information element may include for example a message like "your support need is identified and corresponding service inquiry is under processing in the system, please wait", or it can be a message like "the safety network around you is currently working efficiently, you are not alone, and the system will automatically react if you need help". Those messages illustrate the situational awareness characteristics of the processing system 1005 - not only the current acute situation, but also predicting the future performance with the help of integrated information from multiple sources. In this figure the service elements 1011, 1012, and 1013 are situated outside the processing system 1005 but the processing system may include them. The education system 1013 and the situation awareness processing system 1004 may be the same element.

### Description of the safety ball in graphical form and definition of information processing in it

Figure 1 illustrates a background infrastructure of the SBA, needed to implement it to elderly care practice. Figure 2 shows the "Safety Ball" of SBA in a graphical form. The safety ball 2001 is a virtual surrounding element of processed sensor information items 2003, 2004, and 2005. Behind each of those items there are one or more variables from sensor system 1002, analyzed with related information inside the processing system 1005. Principally the processing is executed by the situation processing element 1003 and it is configured for each elderly person 1001 by using parameters in an individual data storage location 1009. The result of processing is the location of the information item in the framework of the safety ball 2001, following the guidelines:
- If everything is in a good situation regarding the safety and security of the elderly person 1001, location of the corresponding information item is near to the middle point 2002 of the safety ball.
- If the data indicates deviation from the safe situation, the location of the information item begins to deviate from the middle point 2002 accordingly.
- If the data indicates acute danger or substantially increased risk for unwanted situations, the corresponding location of the information item pierces the boundary of the safety ball 2001.

### Sensor independent approach to information processing

The previous processing principle is produced independently by different sensor signals. Therefore, the group of information items forms an easily applicable method to monitor current situation of the elderly people according to the available data. To utilize the SBA in elderly care, the processing system 1005 is configured to continuously monitor situations, as illustrated in Figure 2, and to react according to configurations done. In basic mode for every case where the boundary of safety ball 2001 will be hit, corresponding alarm is executed in processing system 1005 by including the current alarming graph or description about the safety ball e.g. to the information element 1006, which will be sent further to the corresponding responsible nurse on duty of the remote specialist service 1011. In this case, the responsible nurse receives the information element about the current situation and reacts by taking required actions and utilizing the information element 1014 in communication with the elderly person 1001 if needed. Alternatively, the alarm can be sent to the elderly care resource 1016, by using the information element 1015 to relay the safety ball information. The information element 1015 may include also information about timing of the alarm, like the exact time when the boundary of the safety ball 2001 was hit, or required response time for the elderly care resource 1016 to react to the alarm. The timing information is handled by the processing system 1005. The sensitivity and specificity of the system can be altered according to user's need by changing the distance between the boundary of the safety ball 2001 and the middle point 2002. This possibility is important especially because the characteristics and purpose of distinct sensors may vary a lot, even though all the different sensors can be described inside the same safety ball. Similarly, individual elderlies may have highly varying needs of safety in sense of sensitivity and specificity. Due to increasing amount of information it is possible to increase sensitivity and specificity of the analysis, it is possible to create "libraries" about safety ball boundaries from common environments for individual use.

### Different trigger levels to generate actions in the elderly care processes

In safety ball several virtual boundaries can be formed, as exemplified in Figure 2 with boundaries 2006 and 2007. The processing system 1005 can use relative location of information items compared to those boundaries in determination of the best proposed action in alarms, as configured in data storage 1010. For example, the remote expert service 1011 or elderly care resource 1016 can be selected on the basis of severity of the exceptions according to the safety ball and its different boundaries.

### Visualization of safety ball according to time survey

Figure 3 illustrates functioning of SBA in time domain according to current situation processing 1003. The time axis 3006 shows development of safety ball information according to increasing time. The processing system 1005 can be configured to calculate a new safety ball after determined time step 3001. If some information item indicates the increased risk, the situation processing element 1003 locates it outside the safety ball, which releases the alarm, as illustrated in safety ball 3002 in case of information item 3005. The process is automated and the corresponding configuration data is stored in data storage 1010. The idea of SBA in this is to increase cumulative intelligence of the processing system 1005 with the help of this feedback loop. Also, this process is useful to elderly care processes because it simultaneously produces automated documentation about elderly people's status, which enhances the safety assessment and therefore improves quality management.

### Producing situational awareness with the help of safety ball; implementing proactive actions by communication between the safety ball and care process

Figure 4 shows the profound principle of situational awareness processing element 1004 in time domain. This processing element makes predictions about possible future states of the elderly, on the basis of the current safety ball 2001, resulting in a forecast of the next safety ball 4002, and also about the following safety balls as illustrated with safety ball 4004. The forecast of upcoming safety ball includes expectations about information items, where the information items outside the safety ball are important, as illustrated with the information item 4005. The situational awareness processing element 1004 is responsible for producing a proposal about proactive actions for elderly care process shown in Figure 1. In this process the situation awareness processing element 1004 utilizes all available information around or inside the processing system 1005, including e.g. available data in customer information system 1012 or instructions about the proposed actions in education system 1013, or prediction patterns stored in the element 1004, produced in the form of an information set 4006. An example about the information set 4006 is: "On the basis of safety ball analyses the elderly person has an increased risk of falling in the next twenty-four hours. On the basis of medicine information the elderly person's balance in medication requires inspection. The proposed procedure to check the medication balance is shown in the following educational checklist - instructions." Like in the case of Figure 3, also preventive actions and proposals are stored in the data storage 1010 to be used for benefit of quality system of the elderly care.

### Comprehensive standard-like risk management approach integrated to the safety ball, resulting in experience of safe

The comprehensive risk management feature is implemented to SBA by a procedure illustrated in Figure 5; it is defined that every individual elderly person 1001 has his or her own individual risk management table 5000, stored in an individual data storage location 1009. The purpose of this table is to provide information for the processing system 1005 about the parameters, rules and other available required information to execute functionality of SBA as illustrated in Figures 1-4. In this scope two objectives are important; to technically determine distinct risk cases with all the required information, and to conceptually provide safety information for the elderly person 1001 about comprehensive risk management with the help of the risk management table 5000.

The risk management table 5000 includes essentially five columns of information. Those columns describe characteristics of single risks of the elderly person 1001 and their handling in light of general risk management principles. Further, every risk row 5010, 5011, 5012, which are exemplified in Figure 5, states one single risk of the elderly person 1001. The number of risk rows is theoretically large, in order to obtain comprehensive risk management instead of only a selected case-by-case risk analysis. To summarize, the risk management table 5000 with all its columns and rows is an important, comprehensive tool for SBA. In the following the methodology of usage of the risk management table 5000 in SBA is described in more detail.

### Modeling of the risk environment within the extent of parameters available

The risk description column 5001 describes the risk in real life in an easily interpretable form, e.g. "the elderly person falls in the bathroom at night time and is not able to get up without help". Every risk description may have several resolution limited parameters, for example resolution limited parameters 5002, 5003, 5004, 5005 as illustrated in Figure 5. Those parameters draw the extent of the risk management table 5000. The risk description 5001 can be mathematically defined on the basis of resolution limited parameters with the following method, illustrated with an example:
- Let the parameter 5002 have 4 possible states, e.g. "living room", "kitchen", "bedroom", and "toilet", e.g. acquired from indoor positioning and communication (IPCS) based system.
- Let the parameter 5003 have 4 possible states, e.g. "morning", "midday", "afternoon", "night".
- Let the parameter 5004 have 3 possible states, e.g. "person has stayed in the current indoor location 0 minutes", "person has stayed in the current indoor location 10 minutes" and "person has stayed in the current indoor location 60 minutes".
- Let the parameter 5005 have 2 possible states, e.g. "no signs of memory disturbances", "some signs of memory disturbances".

The example shows the meaning of term "resolution limited" of the parameters; every parameter describes a feature of the elderly person 1001, but in the resolution which is defined in the processing system 1005. In the example the number of the possible alternate states is 4 x 4 x 3 x 2 = 96. With the help of this it is possible to fill in the risk management table 5000 with the corresponding 96 rows. The result of this process is a comprehensive risk management table 5000, which is stored in the processing system 1005, to be utilized in elderly care process in the extent of Figure 1. The resolution of the parameters can be made by scaling depending on the needs of the elderly care processes; for some elderly people a very rough level (low resolution) might be enough, but for some others a very detailed resolution might be needed. Further, as illustrated in the example, the source of the parameters may vary; in the example parameters 5002, 5003, and 5004 are related to indoor positioning data, and parameter 5005 is related to customer information system 1012.

The definition of risk descriptions 5001 is modeling and it is done by the situational awareness processing element 1004. This element includes means to provide modeling about possible future states of the elderly. It can use "general libraries" in data storage 1010, e.g. as mentioned with the term SBA library previously, about typical cause-consequence relationships to provide basic modeling for risk management, and further refine the modeling with the help of information in an individual data storage location 1009.

### Communication about modeled risk management configurations to end-users to create experience of preventive safety

It is emphasized that the comprehensive characteristics of the risk management can be informed to the elderly person 1001 with the help of the information element 1014 to provide the elderly with experience of comprehensive risk management, and to support his or her daily life with the feeling that "I'm safe; the safety network around me is efficient".

After modeling of the risk environment of the elderly resulting into a number of rows in the risk management table, corresponding classification is done for them with the risk classification column 5006. If the situation in the row is not dangerous, the classification is "green", and if the situation is clearly dangerous, the "red" classification will be done, and if the situation is something in between, "yellow" classification will be stated. The corresponding numerical index is used to mathematically handle the classification.

Further, according to risk classification row 5006 the preventive action row 5007 is defined if the classification row 5006 indicates that some preventive actions are needed to reach lower total risk status, e.g. to lower the risk status from red to yellow or from yellow to green level. This preventive action row is connected to current situation processing element 1003; principally the preventive actions are the rules which cause piercing of the safety ball 2001 with information items which exist in case of the current risk row. After definition of preventive action the corresponding risk classification after preventive action 5008 is stated with the same procedure as row 5006; the general objective is to try to obtain lower risk classification in the row 5008 compared to the row 5006. Again, it is emphasized that these characteristics of risk management can be informed to the elderly person 1001 with the help of information element 1014 to support his or her experience about the existing safety network, by indicating the processing and work effort which has been paid on configuration of preventive actions.

### How to improve the safety conditions based on safety ball analyzing and automatically alarming procedures

The resource definition column 5009 is used to determine resources which are used in case of realization of the current risk row. This is connected with the processing system 1005, which further communicates with different elderly care resources, e.g. with elderly care resources 1016 or resources by a special service system 1011.

The dynamics of development and utilization of the risk management table 5000 are produced as follows: the situational awareness processing element 1004 is responsible for modeling needed for filling of the risk management table. After risk classification and determination of preventive actions, the risk management table is implemented to daily practice of elderly care by means of current situation processing element 1003 and situational awareness processing element 1004, which further produce cumulative information to the data storage 1010. This implementation is done in co-operation between the expert who has defined the risk management table 5000 and the elderly care organization or the elderly person. Parallel to the risk management table 5000 and the cumulative information in data storage 1010 distinct processes are formulating material to refine the risk management table. For example, if the system produces false-positive alarms, corresponding revision can be done, by using the cumulative information as an argument for the changes to be made. Again, in continuous development the communication with the elderly person 1001 with the help of information element 1014 is used if needed to support continuous characteristics of the experience of his or her safety. Figure 6 exemplifies dynamics of quality information in case of data of a single elderly person; with the help of a short-term date analysis (one week data collection) it is possible to define e.g. acute "abnormal toilet visit" cases with a high accuracy. Further, due to the amount of increased information, it is possible to identify more and more complicated information, even specific quantitative feedback about slowly developing changes in the performance of the elderly person for rehabilitative care purposes, as exemplified in Figure 6.

Figure 7 illustrates the role and benefits of dynamic power of SBA according to the principle of the "swarming concept", presented in Military History of Swarming (2003) by Sean J.A. Edwards. By changing the application topic from military to elderly care, the x axis 7001 illustrates dispersion of the elderly care process system elements and the y axis 7002 illustrates nonlinearity of the system. The nonlinearity and its management have a connection to the performance of the system. The system elements 7003, 7004, 7005, and 7006 are resources (even human or material resources) which can be used to make work in relation to work objective 7008. In the framework of Figure 1 the system elements are in principle distinct elderly care resources 1016 and the objective is the elderly person 1001 with his or her current care needs. The responsibility of the elderly care management is to apply the resources efficiently to successfully handle the work objective 7008.

The block 7007 illustrates a situation where the dispersion of the system elements and work objectives is low, and simultaneously the relating nonlinearity of capacities is low. In this block system elements can reach the work objectives step by step, in a linear and in an easy-to-characterize manner.

Correspondingly, in block 7009 the dispersion of the system is moderate as is the corresponding nonlinearity. The increasing dispersion is caused e.g. because of the non-deterministic variation of the current situation of the system elements and work objectives. From the risk management point of view, perhaps some risks have realized from work management side - all elements are not exactly in their expected places. Also, perhaps the situation of the work objective has changed compared to the initial situation - requiring some fine-tuning for the work which has to be done.

Further, in block 7010 the dispersion of the system and the nonlinearity of the system are continuously high. The situation of system elements and work objective are changing all the time. To reach proper objectives in the work a swarming type of procedure would be beneficial.

The block 7007 illustrates the "ideal" situation in elderly care, including assumption about abundant resources available; the routines are highly established and e.g. the rosters of nurses are easy to define. But, what will happen if the dispersion of the system capabilities increases? This increase may manifest e.g. because of too limited resources of nurses compared to the number elderly people, or because of an increasing amount of unpredictable events among the elderly people. The increasing dispersion causes increase in nonlinearity of the system, as illustrated with the block 7009 where the location (=situation) of elements is not strictly ordered like in block 7007. In this case e.g. rigid shift planning does not work anymore. Simultaneously, the challenge of management of elderly care increases substantially; how to address proper resources to tackle rapidly upcoming varying distinct work objectives? Further, if the situation about high dispersion will continue, the high dispersion in elderly care process, in sense of Figure 1, may develop even into a permanent situation. This situation is exemplified with highly varying locations (=situations) of elements inside the block 7010. Because the ideal case in elderly care resourcing is rarely possible, to manage that kind of situation, the power of swarming - intelligent parallel concentration of abilities of resources - should be released. The SBA form essential elements to establish the swarming in elderly care management; the automated real-time analyzes of data, produced by situational awareness processing element 1004, makes it possible to manage increasing dispersion of the system. Both the elements 1003 and 1004 can include specific tools, e.g. artificial neural networks, which have the ability to effectively manage data processing of nonlinear features, arising from the increase of disperse of the system. The feedback loops, which increase in real-time the information in the data storage 1010, make it possible to produce management - guided swarming in elderly care work. The information elements 1006, 1007, 1008, 1014, and 1015 establish real-time guidance of actions of elderly care resources 1016 to establish swarming effects with no "collisions" between the elements of the system.

### Variations of the possible implementations of the invention

Within the scope of the invention even solutions deviating from the aforedescribed can be considered.

### From single and simple sensors to sensor fusion, ambient sensors and indirect usage of sensor-like information sources

For example, the sensor system 1002 may include overall means for collecting information about incidents which might jeopardize the status and performance of the elderly person 1001. The means might include public databases e.g. about the environment like the outdoor temperature, data which includes information about the current possibility to access health services around the living environment of the elderly person 1001, or restricted access databases about the medication or e-recipies somehow relating to the elderly person 1001, like commonly applied quality and cost-efficacy assessment and monitoring information system RAI®. Therefore the sensor system is not restricted to be tailored only for the safety ball analysis purposes. Also, the communication between the sensor system 1002 and the processing system 1005 may have a number of different protocols; some sensors may send continuous information to the processing system 1005 with a high sample rate, but controversially some other information sources may be transmitted non-periodically, depending on the characteristics of the information source.

### Safety ball for company, safety ball for individual

Principally the SBA is defined for individual elderly people to be further applied in elderly care processes. However, the SBA concept can also be applied in a similar manner to elderly care organizations, too. In this form each elderly care organization would have one SBA, illustrating its comprehensive situation, consisting of information elements of its essential parts. For example, one information element could be the level of available personnel resources and another information element would be the general risk level of all elderly people of the organization. In this SBA the situational awareness processing element 1004 would have an important role in producing proposals for the organization management. The risk management table 5000 could be used with the same analogue also for the organization level; for example preventive actions in case of a sudden lack of care resources could be modeled and handled with the same principle as in the case of a single elderly person. Moreover, the organization level management of resources in a dynamic environment, illustrated in Figure 7, would have a solid connection with the SBA of the organization. In this connection the time-domain perspective of the SBA, as illustrated in Figure 4, creates a tool to manage the "swarming effect" in a complex and nonlinear environment of the work management.

### Transparent user interface methodology; time and place independent user interfaces

User engagement has been mentioned to have some value; in addition to means to communicate between the parties of the SBA by means of information elements 1006, 1007, 1008, 1014, and 1015, also other routes for communication can be used, for example by providing a cloud service based transparent joint access for all resource parties to information which concerns their operation as a part of the elderly care process.

### Three-dimensional graphics for visualization of the safety ball; analogues from computer games

There are multiple alternative possibilities for the visualization of the safety ball. The principle is illustrated in Figures 2, 3, and 4. For example, appropriate vector graphics with user-friendly implementation and user interface provide a lot of means for efficient user interaction. Especially making predictions about the possible future states of the safety ball may be important; how to visualize situational awareness in the most efficient way. For example the elderly care resource 1016 could be provided with predictive information generated by solutions typically used in the graphics of computer games. Similarly, connection between the processing system 1005 and the education system 1013 may include interactive gamification-type exchange about the need of simulations and providing of scenarios, based on real-life data about elderly care, collected to data storage 1010.

### Integration of decision support systems to improve performance of the system

In situational awareness processing, illustrated in Figure 4, different kinds of decision supporting systems could be applied to make proposals about beneficial actions with the information item 4005. In the background of the decision supporting system current science research about the relationship e.g. between signs and symptoms and health problems can be used. Those could be used in the sense of general information which could be stored to the data storage 1010, in co-operation with research institutions. The research done about medical decision supporting systems is eminent. Many scientific findings might be implemented into daily use with the help of SBA.

### Extending risk management level of details with the help of advanced numerical modeling, utilization of artificial intelligence, Reason's model and ISO-standards

Numerical modeling can be used to model risk management table 5000 also in an extended sense as described in Figure 5. The situational awareness processing element 1004 might have an extensive role in evaluating risk management table 5000 automatically row by row to identify inefficient features from the elderly care organization's processes, by investigating a number of different risk tables of elderly people under the same organization. The processes can be configured to independently and periodically analyze data models. If a significant improvement proposal for the elderly care organization can be defined, a system could produce the report and proposal for the elderly care organization. This might produce improvements in operations by improving the projective views for the future by application of the current situation processing element 1003 or for the situational awareness processing element 1004. Further, cloud computing, big data and data mining include possibilities which can be utilized by the situational awareness processing element 1004 to improve risk management table 5000 with the help of the approach without any valid hypothesis from the existing safety threat. According to this "unknown" risk assessment model, the information might produce a cue or an image of exceptional findings with the help of self-organizing map (SOM) artificial networks, having abilities to manage data analyzes without exact hypothesis. This kind of an ability might be valuable in identifying risky situations from the risk management table 5000 in the sense of a so-called Reason's model about human errors. The improvement of risk management table 5000 with those tools could be integrated into systematic risk management procedures presented e.g. in ISO 14901 standard. The continuous information flow from the sensor system 1002, connected with the help of the previously described analyzes, including risk management table 5000 and its relations with the Reason's model and ISO standard type approach, may form a substantial element about dynamical development of overall quality of elderly care process, illustrated in Figure 1.

### Extending time domain analyses of risk management with the help of integrative analysis of relationships

The time domain of risk management can be taken into account with the risk management table 5000, by connecting the time domain of SBA as illustrated in Figures 3 and 4 to it in a case-by-case manner. However, the time domain analyses can be extended with the help of integrative analysis to cover all possible changes in the SBA according to time. For example, an increasing trend during at least three consecutive days in a specific information element of the SBA could be identified and defined to produce an alarm according to the principle of the SBA, even though the corresponding information element does not pierce the boundary of the safety ball. This example illustrates the power of feature extraction of information due to time to find hidden new information for the benefit of the elderly care purposes. Further, a comprehensive time domain changes analysis can be defined with the help of integrative logic, which includes all possible combinations in variations of the information elements according to the time. Those combinations can be calculated with the help of an appropriate mathematical modeling. The extension of the analysis in the time domain increases the possibilities for handling risks especially relating to slowly developing changes in the performance of the elderly people.

### Application of artificial intelligence models to increase accuracy of proactivity and to produce simulation scenarios for education and training purposes

The role of artificial intelligence algorithms (artificial neural networks, genetic algorithms, fuzzy logic, or their combinations) can also be increased in a method level by making an artificial copy of all elements of the elderly care processes in Figure 1, resulting in a kind of a "virtual or artificial second life" of the real elderly care process. It is important to remember that improving and managing working processes are crucially important in improving the cost-effectiveness in elderly care and on the flipside, in reducing human errors. In this copy an independent or semi-independent artificial intelligent module is used to model status and actions of the elderly person 1001, the elderly care resource 1016, and the resources in the elements 1011, 1012, and 1013. An individual artificial intelligent module could be used for every elderly person of the care process and for every elderly care resource of the process. The data into data storage 1010 of the real elderly care process could be used to train the artificial intelligence modules to work properly. For example, daily routines of the elderly person could be predicted on the basis of the previous data, and typical reactions of the elderly care resource to a specific piece of information in the element 1015 could be predicted on the basis of the history data about the documented related actions. The increase of cumulative data will lead into more and more accurate operation of the artificial copy, imitating the operation of the real elderly care process. After the modeling accuracy of the artificial copy reaches the required level, it can be used to further develop the processing elements 1003 and 1004. Especially, the model can be used to produce predictions about future states of SBA in the sense of Figure 4. Moreover, the artificial modeling can be utilized in interaction with the education system 1013, by providing it with scenarios already found in the real elderly care processes, but also with upcoming possible situations which might need proactive education or training.

### Optimal resource usage

The operation of SBA produces cumulative information to the data storage 1010. The typical actions in elderly care due to specific situations may form statistical clusters of situations, which illustrate characteristics of the work processes. Analyses of those events could be included into the situational awareness processing element 1004 in order to produce proposals about optimization of resource usage according to the reached cumulative experience. Further, the proposal about optimal usage of resources, relating to the same cumulative experience, could be included to be a part of the future predictions of SBA, in a sense illustrated in Figure 4.

### Extending application of the SBA to other target groups

Finally, even though elderly care has been mentioned as an application field of the SBA, the same method could be applied for example among other target groups, like disabled people, in rehabilitation care overall, or even among the normal population healthcare. On a more general level the SBA could be applied overall in the social and healthcare processes. The most efficient the SBA would be in systems which have measurable processes. Moreover, the SBA would be beneficial also to be applied in industry where safety and cost-effectiveness are important.

It will be stated that there are many structural similarities in elderly care systems with other complicated and dynamic systems where the Ensley's SA theory and other safety assessment principles used in complex working environment can be applied. Comparing e.g. the basics of flight safety where the history of continuous improvement of safety and security processes have been going on, the same kind of procedures can be applied to elderly care producing safety and security for ageing people. The SBA model is an innovative tool for those purposes.

Some preferred embodiments of the invention are described above. The invention, however, is not limited to the previously described embodiments. The inventive idea can be applied in several ways within the limits of the claims.

## Claims

1. A system for monitoring safety in elderly care, where the system comprises a sensor system (1002), which is configured to monitor at least one elderly person (1001), a processing system (1005) comprising a data storage (1010), which processing system is configured to collect, process, and store the data which said sensor system (1002) has collected, which data includes at least time and location, wherein
- the processing system (1005) further comprises
- an individual data storage location (1009) that is configured to store data collected from an individual elderly person,
- a current situation processing element (1003) which is configured to analyze the current situation of the elderly person (1001) based on the information available in the data storage (1010), and to produce variables that describe the status of the elderly person (1001),
- a situation awareness processing element (1004) which is configured to store prediction pattern or patterns and to read said collected data and said prediction patterns and form an information set or sets (4006) based on them and predict upcoming situations of the elderly person (1001) based on the information in the individual data storage location (1009) and the information set or sets, and to produce variables that describe the future status of the elderly person (1001), and
- the processing system (1005) is configured to calculate one or more information items (2003, 2004, 2005; 3005; 4005) based on the variables produced by the current situation processing element (1003) or the situation awareness processing element (1004) or both, which each information item corresponds a risk value,
wherein an individual risk management table (5000) is stored in the individual data storage location (1009) and the individual risk management table contains at least a risk description column (5001) having one or more risk descriptions and to each risk description is connected one or more resolution limited parameters (5002, 5003, 5004, 5005) and each resolution limited parameter has several states, and said individual risk management table is used to calculate the risk values of the information items (2003, 2004, 2005; 3005; 4005), and the processing system (1005) is configured to give an alarm if the risk value of one information item exceeds some predetermined value, and
- the system further comprises an information element (1014) which provides the elderly person (1001) with information about the care process around him/her and the information is based on the calculations of the processing system (1005).

2. The system according to claim 1, **characterized in that** the information items (2003, 2004, 2005; 3005; 4005) having approximately the same time form a group, and the processing system (1005) is configured to form a ball-shaped visual presentation (2001; 3002, 3004; 4002, 4004) based on said group where the information items having a low risk value are situated near the center (2002) of the said ball, the information items having an increased risk value are situated to deviate from the center of the ball, and the information items (4005) causing the alarm are situated outside of the boundary of the ball.

3. The system according to claim 2, **characterized in that** the balls (2001; 3002, 3004; 4002, 4004) with different times are displayed sequentially on a time axis (3006).

4. The system according to claim 2 or 3, **characterized in that** the balls based on the information items based on the variables produced by the situation awareness processing element (1004) describe a future prediction of the safety of the elderly person (1001).

5. The system according to claim 1, **characterized in that** the resolution limited parameters are configured to be scaled by the safety needs of the individual elderly person (1001).

6. The system according to any of the claims 1-5, **characterized in that** the information produced by the information element (1014) is a message that describes the situational awareness characteristics of the processing system (1005) which situational awareness characteristics are related to one or more information items (2003, 2004, 2005; 3005; 4005).

7. The system according to any of the claims 1-6, **characterized in that** the information indicates a risk level of the elderly person (1001) or an action the system has executed or will execute or both the risk level and the action and the risk level is calculated from risk value.

8. The system according to any of the claims 1-7, **characterized in that** the information indicates that the situation of the elderly person is normal.

9. A method for monitoring safety in elderly care, which method uses a system that comprises a sensor system (1002), which is configured to monitor at least one elderly person (1001), a processing system (1005) comprising a data storage (1010), which processing system is configured to collect, process, and store the data which said sensor system (1002) has collected, which data includes at least time and location, wherein the system further comprises an information element (1014) and the method comprises steps where
- the sensor system monitors the elderly person (1001) and gathers data,
- the processing system produces variables that describe the status of the elderly person (1001) based on the data the sensor system has gathered,
- the processing system (1005) calculates one or more information items (2003, 2004, 2005; 3005; 4005) based on the variables produced and each information item corresponds to a risk value, wherein
- an individual risk management table (5000) is stored in the individual data storage location (1009) and the individual risk management table contains at least a risk description column (5001) having one or more risk descriptions and to each risk description is connected one or more resolution limited parameters (5002, 5003, 5004, 5005) and each resolution limited parameter has several states, and said individual risk management table is used to calculate the risk values of the information items (2003, 2004, 2005; 3005; 4005), and
- if any of the risk values exceeds some predetermined value, the processing system gives an alarm, and
- the information element (1014) provides the elderly person (1001) with information about the care process around him/her.

10. The method according to claim 9, **characterized in that** the information items having approximately the same time are displayed as a group and the processing system (1005) forms a ball-shaped visual presentation where the boundary of the ball represents the predetermined value that triggers the alarm and the information items having a low risk value are situated near the center (2002) of the said ball, the information items having an increased risk value are situated to deviate from the center of the ball, and the information items (4005) causing the alarm are situated outside of the boundary of the ball.

11. The method according to claim 9 or 10, **characterized in that** in the system has been stored a prediction pattern or patterns and an information set or sets (4006) are formed based on said prediction pattern or patterns and the data the sensor system has gathered, and the processing system produces variables that describe the future status of the elderly person (1001), and based on these variables predictive information items are calculated.

12. The method according to any of the claims 9-11, **characterized in that** the information element (1014) produces a message that describes the situational awareness characteristics of the processing system (1005) which situational awareness characteristics are related to one or more information items (2003, 2004, 2005; 3005; 4005).

13. The method according to any of the claims 9-12, **characterized in that** the information provided by the information element (1014) indicates at least that the alarm is sent.

14. A computer software product, **characterized in that** it comprises computer program code means stored on a computer-readable storage means, which code means are configured to perform all the steps of the method defined in claims 9-13 when said program is run on a computer.

## Patentansprüche

1. System zum Überwachen der Sicherheit in der Altenpflege, wobei das System ein Sensorsystem (1002), das dazu ausgelegt ist, mindestens eine ältere Person (1001) zu überwachen, ein Verarbeitungssystem (1005) umfasst, das einen Datenspeicher (1010) umfasst, wobei das Verarbeitungssystem dazu ausgelegt ist, die Daten, die vom Sensorsystem (1002) gesammelt wurden, zu sammeln, zu verarbeiten und zu speichern, wobei die Daten mindestens Zeit und Ort beinhalten, wobei
- das Verarbeitungssystem (1005) ferner Folgendes umfasst
- einen einzelnen Datenspeicherort (1009), der dazu ausgelegt ist, Daten, die über eine einzelne ältere Person gesammelt wurden, zu speichern,
- ein Verarbeitungselement (1003) für die aktuelle Situation, das dazu ausgelegt ist, die aktuelle Situation der älteren Person (1001) auf Basis der Informationen, die im Datenspeicher (1010) verfügbar sind, zu analysieren und Variablen zu produzieren, die den Status der älteren Person (1001) beschreiben,
- ein Situationsbewusstseinsverarbeitungselement (1004), das dazu ausgelegt ist, ein oder mehrere Vorhersagemuster zu speichern und die gesammelten Daten und die Vorhersagemuster zu lesen und auf Basis von diesen einen oder mehrere Informationssätze (4006) zu bilden und auf Basis der Informationen am einzelnen Datenspeicherort (1009) und des Informationssatzes bzw. der Informationssätze bevorstehende Situationen der älteren Person (1001) vorherzusagen sowie Variablen, die den künftigen Status der älteren Person (1001) beschreiben, zu produzieren, und
- das Verarbeitungssystem (1005) dazu ausgelegt ist, auf Basis der Variablen, die vom Verarbeitungselement (1003) für die aktuelle Situation oder vom Situationsbewusstseinsverarbeitungselement (1004) oder von beiden produziert wurden, eine oder mehrere Informationskomponenten (2003, 2004, 2005; 3005; 4005) zu berechnen, wobei jede Informationskomponente einem Risikowert entspricht,
wobei eine einzelne Risikoverwaltungstabelle (5000) am einzelnen Datenspeicherort (1009) gespeichert ist und die einzelne Risikoverwaltungstabelle mindestens eine Risikobeschreibungsspalte (5001) mit einer oder mehreren Risikobeschreibungen enthält und mit jeder Risikobeschreibung ein oder mehrere auflösungsbegrenzte Parameter (5002, 5003, 5004, 5005) verbunden sind und jeder auflösungsbegrenzte Parameter mehrere Zustände aufweist und die einzelne Risikoverwaltungstabelle verwendet wird, um die Risikowerte der Informationskomponenten (2003, 2004, 2005; 3005; 4005) zu berechnen, und das Verarbeitungssystem (1005) dazu ausgelegt ist, einen Alarm auszugeben, wenn der Risikowert einer Informationskomponente einen vorbestimmten Wert überschreitet, und
- das System ferner ein Informationselement (1014) umfasst, das der älteren Person (1001) Informationen über den Pflegeprozess um sie bereitstellt und die Informationen auf den Berechnungen des Verarbeitungssystems (1005) basieren.

2. System nach Anspruch 1, **dadurch gekennzeichnet, dass** die Informationskomponenten (2003, 2004, 2005; 3005; 4005), die ungefähr dieselbe Zeit aufweisen, eine Gruppe bilden und das Verarbeitungssystem (1005) dazu ausgelegt ist, auf Basis der Gruppe eine kugelförmige visuelle Präsentation (2001; 3002, 3004; 4002, 4004) zu bilden, wobei die Informationskomponenten mit einem geringen Risikowert in der Nähe der Mitte (2002) der Kugel positioniert sind und die Informationskomponenten mit einem erhöhten Risikowert derart positioniert sind, dass sie von der Mitte der Kugel abweichen, und die Informationskomponenten (4005), die den Alarm verursachen, außerhalb der Grenze der Kugel positioniert sind.

3. System nach Anspruch 2, **dadurch gekennzeichnet, dass** die Kugeln (2001; 3002, 3004; 4002, 4004) mit unterschiedlichen Zeiten sequenziell auf einer Zeitachse (3006) angezeigt werden.

4. System nach Anspruch 2 oder 3, **dadurch gekennzeichnet, dass** die Kugeln, die auf den Informationskomponenten basieren, die auf den Variablen basieren, die vom Situationsbewusstseinsverarbeitungselement (1004) produziert werden, eine künftige Vorhersage der Sicherheit der älteren Person (1001) beschreiben.

5. System nach Anspruch 1, **dadurch gekennzeichnet, dass** die auflösungsbegrenzten Parameter dazu ausgelegt sind, durch die Sicherheitsbedürfnisse der einzelnen älteren Person (1001) skaliert zu werden.

6. System nach einem der Ansprüche 1-5, **dadurch gekennzeichnet, dass** die Information, die vom Informationselement (1014) produziert wird, eine Nachricht ist, die die Situationsbewusstseinscharakteristika des Verarbeitungssystems (1005) beschreibt, wobei die Situationsbewusstseinscharakteristika auf eine oder mehrere Informationskomponenten (2003, 2004, 2005; 3005; 4005) bezogen sind.

7. System nach einem der Ansprüche 1-6, **dadurch gekennzeichnet, dass** die Informationen eine Risikostufe der älteren Person (1001) oder eine Aktion, die das System ausgeführt hat oder ausführen wird, oder sowohl die Risikostufe als auch die Aktion anzeigen und die Risikostufe aus dem Risikowert berechnet wird.

8. System nach einem der Ansprüche 1-7, **dadurch gekennzeichnet, dass** die Informationen anzeigen, dass die Situation der älteren Person normal ist.

9. Verfahren zum Überwachen der Sicherheit in der Altenpflege, wobei das Verfahren ein System verwendet, das ein Sensorsystem (1002), das dazu ausgelegt ist, mindestens eine ältere Person (1001) zu überwachen, ein Verarbeitungssystem (1005) umfasst, das einen Datenspeicher (1010) umfasst, wobei das Verarbeitungssystem dazu ausgelegt ist, die Daten, die vom Sensorsystem (1002) gesammelt wurden, zu sammeln, zu verarbeiten und zu speichern, wobei die Daten mindestens Zeit und Ort beinhalten, wobei das System ferner ein Informationselement (1014) umfasst und das Verfahren Schritte umfasst, wobei
- das Sensorsystem die ältere Person (1001) überwacht und Daten erfasst,
- das Verarbeitungssystem Variablen produziert, die den Status der älteren Person (1001) auf Basis der Daten, die das Sensorsystem erfasst hat, beschreibt,
- das Verarbeitungssystem (1005) auf Basis der produzierten Variablen eine oder mehrere Informationskomponenten (2003, 2004, 2005; 3005; 4005) berechnet und jede Informationskomponente einem Risikowert entspricht, wobei
- eine einzelne Risikoverwaltungstabelle (5000) am einzelnen Datenspeicherort (1009) gespeichert ist und die einzelne Risikoverwaltungstabelle mindestens eine Risikobeschreibungsspalte (5001) mit einer oder mehreren Risikobeschreibungen enthält und mit jeder Risikobeschreibung ein oder mehrere auflösungsbegrenzte Parameter (5002, 5003, 5004, 5005) verbunden sind und jeder auflösungsbegrenzte Parameter mehrere Zustände aufweist und die einzelne Risikoverwaltungstabelle verwendet wird, um die Risikowerte der Informationskomponenten (2003, 2004, 2005; 3005; 4005) zu berechnen, und
- wenn einer der Risikowerte einen vorbestimmten Wert überschreitet, das Verarbeitungssystem einen Alarm ausgibt und
- das Informationselement (1014) der älteren Person (1001) Informationen über den Pflegeprozess um sie bereitstellt.

10. Verfahren nach Anspruch 9, **dadurch gekennzeichnet, dass** die Informationskomponenten, die ungefähr dieselbe Zeit aufweisen, als eine Gruppe angezeigt werden und das Verarbeitungssystem (1005) eine kugelförmige visuelle Präsentation bildet, wobei die Grenze der Kugel den vorbestimmten Wert repräsentiert, der den Alarm auslöst, und die Informationskomponenten mit einem geringen Risikowert in der Nähe der Mitte (2002) der Kugel positioniert sind und die Informationskomponenten mit einem erhöhten Risikowert derart positioniert sind, dass sie von der Mitte der Kugel abweichen, und die Informationskomponenten (4005), die den Alarm verursachen, außerhalb der Grenze der Kugel positioniert sind.

11. Verfahren nach Anspruch 9 oder 10, **dadurch gekennzeichnet, dass** im System ein oder mehrere Vorhersagemuster gespeichert wurden und ein oder mehrere Informationssätze (4006) auf Basis des einen oder der mehreren Vorhersagemuster und der Daten, die das Sensorsystem erfasst hat, gebildet werden und das Verarbeitungssystem Variablen produziert, die den künftigen Status der älteren Person (1001) beschreiben, und auf Basis dieser Variablen Vorhersageinformationskomponenten berechnet werden.

12. Verfahren nach einem der Ansprüche 9-11, **dadurch gekennzeichnet, dass** das Informationselement (1014) eine Nachricht produziert, die die Situationsbewusstseinscharakteristika des Verarbeitungssystems (1005) beschreibt, wobei die Situationsbewusstseinscharakteristika auf eine oder mehrere Informationskomponenten (2003, 2004, 2005; 3005; 4005) bezogen sind.

13. Verfahren nach einem der Ansprüche 9-12, **dadurch gekennzeichnet, dass** die Informationen, die vom Informationselement (1014) bereitgestellt werden, mindestens anzeigen, dass der Alarm gesendet wurde.

14. Computersoftwareprodukt, **dadurch gekennzeichnet, dass** es Computerprogrammcodemittel auf einem computerlesbaren Speichermittel umfasst, wobei das Codemittel dazu ausgelegt ist, alle Schritte des Verfahrens, das in den Ansprüchen 9-13 definiert ist, durchzuführen, wenn das Programm auf einem Computer läuft.

## Revendications

1. Système permettant de surveiller la sécurité lors de soins aux personnes âgées, où le système comprend un système de capteurs (1002), qui est configuré pour surveiller au moins une personne âgée (1001), un système de traitement (1005) comprenant un stockage de données (1010), ce système de traitement étant configuré pour collecter, traiter, et stocker les données que ledit système de capteurs (1002) a collectées, ces données incluant au moins un temps et un emplacement, dans lequel
- le système de traitement (1005) comprend en outre
- un emplacement de stockage de données individuelles (1009) qui est configuré pour stocker des données collectées à partir d'une personne âgée individuelle,
- un élément de traitement de situation actuelle (1003) qui est configuré pour analyser la situation actuelle de la personne âgée (1001) sur la base des informations disponibles dans le stockage de données (1010), et pour produire des variables qui décrivent le statut de la personne âgée (1001),
- un élément de traitement de sensibilisation à une situation (1004) qui est configuré pour stocker un schéma ou des schémas de prévision et pour lire lesdites données collectées et lesdits schémas de prévision et former un ensemble ou des ensembles d'informations (4006) basés sur ceux-ci et prédire des situations potentielles de la personne âgée (1001) sur la base des informations dans l'emplacement de stockage de données individuelles (1009) et de l'ensemble ou des ensembles d'informations, et pour produire des variables qui décrivent le statut futur de la personne âgée (1001), et
- le système de traitement (1005) est configuré pour calculer un ou plusieurs articles d'information (2003, 2004, 2005 ; 3005 ; 4005) sur la base des variables produites par l'élément de traitement de situation actuelle (1003) ou l'élément de traitement de sensibilisation à une situation (1004) ou les deux, chaque article d'information correspondant à une valeur de risque,
dans lequel un tableau de gestion de risques individuels (5000) est stocké dans l'emplacement de stockage de données individuelles (1009) et le tableau de gestion de risques individuels contient au moins une colonne de description de risques (5001) chacune ayant une ou plusieurs descriptions de risque et à chaque description de risque sont connectés un ou plusieurs paramètres limités de résolution (5002, 5003, 5004, 5005) et chaque paramètre limité de résolution a plusieurs états, et ledit tableau de gestion de risques individuels possède plusieurs états, et ledit tableau de gestion de risques individuels est utilisé pour calculer les valeurs de risque des articles d'information (2003, 2004, 2005 ; 3005 ; 4005), et le système de traitement (1005) est configuré pour donner une alarme si la valeur de risque d'un article d'information dépasse une certaine valeur prédéterminée, et
- le système comprend en outre un élément d'information (1014) qui fournit à la personne âgée (1001) des informations au sujet du processus de soins autour d'elle et les informations étant basées sur les calculs du système de traitement (1005).

2. Système selon la revendication 1, **caractérisé en ce que** les articles d'information (2003, 2004, 2005 ; 3005 ; 4005) ayant approximativement le même temps forment un groupe, et le système de traitement (1005) est configuré pour former une présentation visuelle à formes en boules (2001 ; 3002, 3004 ; 4002, 4004) sur la base dudit groupe où les articles d'information ayant une valeur de risque faible sont situés près du centre (2002) de ladite boule, les articles d'information ayant une valeur de risque accru sont situés pour être déviés à partir du centre de la boule, et les articles d'information (4005) provoquant l'alarme étant situés à l'extérieur de la limite de la boule.

3. Système selon la revendication 2, **caractérisé en ce que** les boules (2001 ; 3002, 3004 ; 4002, 4004) avec des temps différents sont affichées de manière séquentielle sur un axe des temps (3006).

4. Système selon la revendication 2 ou 3, **caractérisé en ce que** les boules basées sur les articles d'information basés sur les variables produites par l'élément de traitement de sensibilisation à une situation (1004) décrivent une prévision future de la sécurité de la personne âgée (1001).

5. Système selon la revendication 1, **caractérisé en ce que** les paramètres limités de résolution sont configurés pour être mis à l'échelle par les besoins en sécurité de la personne âgée (1001).

6. Système selon n'importe laquelle des revendications 1 à 5, **caractérisé en ce que** les informations produites par l'élément d'information (1014) est un message qui décrit les caractéristiques de sensibilisation en situation du système de traitement (1005), ces caractéristiques de sensibilisation en situation étant liées à un ou plusieurs articles d'information (2003, 2004, 2005 ; 3005 ; 4005).

7. Système selon n'importe laquelle des revendications 1 à 6, **caractérisé en ce que** les informations indiquent un niveau de risque de la personne âgée (1001) ou une action que le système a exécutée ou va exécuter ou à la fois le niveau de risque et l'action et le niveau de risque est calculé à partir d'une valeur de risque.

8. Système selon n'importe laquelle des revendications 1 à 7, **caractérisé en ce que** les informations indiquent que la situation de la personne âgée est normale.

9. Procédé permettant de surveiller la sécurité lors de soins aux personnes âgées, ce procédé utilisant un système qui comprend un système de capteurs (1002), qui est configuré pour surveiller au moins une personne âgée (1001), un système de traitement (1005) comprenant un stockage de données (1010), ce système de traitement étant configuré pour collecter, traiter, et stocker les données que ledit système de capteurs (1002) a collectées, ces données incluant au moins un temps et un emplacement, dans lequel le système comprend en outre un élément d'information (1014) et le procédé comprend des étapes où
- le système de capteurs surveille la personne âgée (1001) et recueille des données,
- le système de traitement produit des variables qui décrivent le statut de la personne âgée (1001) sur la base des données que le système de capteurs a recueillies,
- le système de traitement (1005) calcule un ou plusieurs articles d'information (2003, 2004, 2005 ; 3005 ; 4005) sur la base des variables produites et chaque article d'information correspond à une valeur de risque, dans lequel
- un tableau de gestion de risques individuels (5000) est stocké dans l'emplacement de stockage de données individuelles (1009) et le tableau de gestion de risques individuels contient au moins une colonne de description de risques (5001) chacune ayant une ou plusieurs descriptions de risque et à chaque description de risque sont connectés un ou plusieurs paramètres limités de résolution (5002, 5003, 5004, 5005) et chaque paramètre limité de résolution a plusieurs états, et ledit tableau de gestion de risques individuels possède plusieurs états, et ledit tableau de gestion de risques individuels est utilisé pour calculer les valeurs de risque des articles d'information (2003, 2004, 2005 ; 3005 ; 4005), et
- si l'une quelconque des valeurs de risque dépasse une certaine valeur prédéterminée, le système de traitement donne une alarme, et
- l'élément d'information (1014) fournit à la personne âgée (1001) des informations au sujet du processus de soins autour d'elle.

10. Procédé selon la revendication 9, **caractérisé en ce que** les articles d'information ayant approximativement le même temps sont affichés en tant que groupe et le système de traitement (1005) forme une présentation visuelle à formes en boules où la limite de la boule représente la valeur prédéterminée qui déclenche l'alarme et les articles d'information ayant une valeur de risque faible sont situés près du centre (2002) de ladite boule, les articles d'information ayant une valeur de risque accru sont situés pour être déviés à partir du centre de la boule, et les articles d'information (4005) provoquant l'alarme sont situés à l'extérieur de la limite de la boule.

11. Procédé selon la revendication 9 ou 10, **caractérisé en ce que** dans le système ont été stockés un schéma ou des schémas de prévision et un ensemble ou des ensembles d'information (4006) sont formés sur la base dudit schéma ou desdits schémas de prévision et des données que le système de capteurs a recueillies, et le système de traitement produit des variables qui décrivent le statut futur de la personne âgée (1001), et les articles d'information prédictifs sont calculés sur la base de ces variables.

12. Procédé selon n'importe laquelle des revendications 9 à 11, **caractérisé en ce que** l'élément d'information (1014) produit un message qui décrit les caractéristiques de sensibilisation en situation du système de traitement (1005), ces caractéristiques de sensibilisation en situation étant liées à un ou plusieurs articles d'information (2003, 2004, 2005 ; 3005 ; 4005).

13. Procédé selon n'importe laquelle des revendications 9 à 12, **caractérisé en ce que** les informations fournies par l'élément d'information (1014) indiquent au moins que l'alarme est envoyée.

14. Produit à logiciel informatique, **caractérisé en ce qu'**il comprend des moyens de code de programme informatique stockés sur un moyen de stockage lisible par ordinateur, ces moyens de code étant configurés pour réaliser toutes les étapes du procédé défini dans les revendications 9 à 13 lorsque ledit programme est exploité sur un ordinateur.
